# FASCICULE DE BREVET EUROPEEN

(11) **EP 1 802 446 B1**
(45) Date de publication et mention de la délivrance du brevet: **12.12.2007**
(21) Numéro de dépôt: 05809266.9
(22) Date de dépôt: 18.10.2005
(51) Int. Cl.: B29C 49/78

(54) **PROCEDE DE MESURE CAPACITIVE D'UNE CARACTERISTIQUE D'UN RECIPIENT THERMOPLASTIQUE DANS UN MOULE, MOULE AINSI EQUIPE.**
VERFAHREN ZUR KAPAZITIVEN MESSUNG EINER EIGENSCHAFT EINES THERMOPLASTISCHEN BEHÄLTERS IN EINER GUSSFORM UND DAMIT AUSGERÜSTETE GUSSFORM
METHOD FOR CAPACITIVE MEASUREMENT OF ONE CHARACTERISTIC OF A THERMOPLASTIC CONTAINER IN A MOLD, AND MOLD EQUIPPED WITH SAME

(30) Priorité: 21.10.2004 FR 0411220
(43) Date de publication de la demande: 04.07.2007
(73) Titulaire: Sidel Participations, 76930 Octeville Sur Mer (FR)
(72) Inventeur: FEUILLOLEY, Guy, c/o Sidel Participations, F-76930 Octeville sur Mer (FR)
(74) Mandataire: Gorrée, Jean-Michel
(86) Numéro de dépôt international: PCT/FR2005/002585
(87) Numéro de publication internationale: WO 2006/042960

(56) Documents cités:
- EP-A- 0 491 545
- EP-A- 0 544 022
- EP-A- 1 175 990
- US-A- 2 616 068
- PATENT ABSTRACTS OF JAPAN vol. 1996, no. 05, 31 mai 1996 (1996-05-31) & JP 08 025464 A (KYOTO JUSHI SEIKO KK), 30 janvier 1996 (1996-01-30)

## Description

La présente invention concerne des perfectionnements apportés dans le domaine de la fabrication de récipients, notamment de bouteilles, en matériau thermoplastique tel que le PET par un processus de soufflage ou d'étirage-soufflage d'ébauches, notamment de préformes.

Les récipients en matériau thermoplastique présentent des caractéristiques physiques prédéterminées qui sont tributaires des conditions de déroulement du processus de fabrication. Toute dérive des paramètres de fabrication peut entraîner une modification de ces caractéristiques physiques susceptible de conduire finalement à des récipients non exploitables.

Notamment, les récipients en matériau thermoplastique présentent des épaisseurs de paroi qui sont différenciées selon les zones du récipient (corps, épaule, fond notamment), ces épaisseurs étant déterminées pour que chacune des zones présente une résistance mécanique appropriée tout en faisant en sorte que ces épaisseurs soient minimales pour économiser la quantité de matière première utilisée.

Toutefois, les conditions de fonctionnement de l'installation peuvent se modifier en cours de fonctionnement pour des raisons diverses et un ou plusieurs paramètres du processus de moulage peuvent subir des dérives de telle sorte que des récipients ainsi obtenus possèdent, localement ou dans leur ensemble, une paroi dont l'épaisseur n'est pas conforme au cahier des charges, en d'autres termes des récipients dont au moins certaines zones de parois sont trop épaisses et/ou d'autres zones trop minces, d'autres zones encore pouvant être d'épaisseur correcte. Une telle non-conformité n'est pas acceptable : en effet, la paroi est affaiblie dans les zones où elle est trop mince ; en outre, la quantité de matière des préformes est optimisée pour que le profil d'épaisseur du récipient moulé réponde au cahier des charges, de sorte que si certaines zones du récipient moulé sont trop épaisses, c'est en fait parce que de la matière qui aurait dû se trouver dans une ou des zones avoisinantes est venue aider à la constitution des zones trop épaisses, donc au détriment desdites zones avoisinantes qui, elles, sont donc plus minces qu'elles ne le devraient et sont alors affaiblies.

De même, la densité, qui est représentative du taux de cristallinité du matériau constitutif du récipient, conditionne l'aptitude du récipient à supporter sans déformation notable des contraintes thermiques (pasteurisation, remplissage à chaud) ou de pression (liquides carbonatés). Toute dérive de la densité, donc du taux de cristallinité, sous l'influence de modification(s) des paramètres du processus de moulage peut conduire à des récipients impropres à supporter les contraintes prévues.

Des contrôles peuvent certes être effectués sur les récipients terminés. Toutefois, pour le contrôle de l'épaisseur, il s'agit actuellement de contrôles effectués à l'aide de systèmes de vision sur des récipients circulant souvent à vitesse élevée. Or, ces contrôles ont lieu dans une zone proche de la sortie de la machine et nécessitent un appareillage susceptible de suivre la cadence de sortie de la machine (typiquement quelques dizaines de milliers de récipients par heure, avec les machines de la Demanderesse) : on ne connaît pas actuellement d'appareillages suffisamment rapides et précis pour détecter des dérives trop faibles sur les caractéristiques mesurées à de telles cadences.

D'après le document US 2,616,068, on connaît un procédé de contrôle de l'épaisseur d'une paroi, notamment d'un récipient à col étroit, en un matériau diélectrique, selon lequel un capteur capacitif est placé en contact avec le matériau diélectrique afin de mesurer la variation de la capacité électrostatique, le dispositif de contrôle étant placé le long d'une ligne de transfert des parois, notamment des récipients.

Des contrôles plus complets sont également possibles. Mais il s'agit alors de contrôles menés sur des récipients prélevés ponctuellement dans la production et les résultats sont obtenus a posteriori alors qu'un très grand nombre de récipients défectueux a pu être produit.

On ajoutera à ces considérations le fait que le défaut de moyens efficaces de contrôles en ligne et en temps réel de la conformité des récipients fabriqués, et notamment de l'épaisseur de leur paroi, conduit à prévoir une marge de sécurité dans la prédétermination de l'épaisseur de paroi des récipients. Cela se traduit par la mise en oeuvre, pour chaque récipient, d'une quantité de matière première qui excède le strict nécessaire. Compte tenu du prix toujours croissant de la matière première telle que le PET au moins dans certaines parties du monde, il apparaît comme souhaitable de pouvoir mieux maîtriser la quantité unitaire de matière première utilisée, et donc notamment de mieux contrôler l'épaisseur des récipients fabriqués.

L'invention vise à apporter une solution au problème posé et à proposer des moyens donnant la possibilité de réaliser un contrôle effectif d'au moins une caractéristique de chaque récipient fabriqué, de telle sorte que l'apparition d'une dérive de cette caractéristique puisse être détectée instantanément ou très rapidement et que le fonctionnement de l'installation de moulage puisse être adapté en conséquence sans retard, afin de réduire à un minimum le nombre des récipients non conformes.

A ces fins, selon un premier de ses aspects l'invention propose un procédé de mesure d'au moins une caractéristique physique de récipients, notamment de bouteilles, fabriqués par soufflage ou étirage-soufflage d'ébauches en matière thermoplastique, notamment en PET, dans un moule comportant au moins une cavité de moulage, caractérisé en ce que ladite mesure est faite par détection d'un effet capacitif ou d'une variation d'effet capacitif dans le moule au cours du soufflage ou de l'étirage-soufflage.

Le procédé de l'invention peut trouver une application particulièrement préférée pour contrôler l'épaisseur de la paroi du récipient en au moins un emplacement de celui-ci, ou encore pour contrôler la densité du matériau de la paroi du récipient en au moins un emplacement de celui-ci (étant rappelé que la densité est représentative du taux de cristallinité du matériau).

De façon pratique pour obtenir une précision optimale de la mesure, on prévoit que la mesure est faite entre la fin du soufflage ou étirage-soufflage et le début de l'ouverture du moule, alors que la paroi du récipient est en contact étroit avec la surface de moulage de la cavité de moulage.

Ainsi, l'invention permet de s'affranchir des limitations dues aux cadences machines, telles qu'évoquées ci avant : en effet, en réalisant la mesure dans le moule on dispose d'un temps suffisant pour réaliser la mesure. Pour fixer les idées, pour effectuer une mesure de l'épaisseur de la paroi du récipient, la mise en oeuvre du procédé conforme à l'invention confère un laps de temps de l'ordre de cinq à dix fois supérieur à celui dont on dispose en recourant à un système de vision.

Selon un deuxième de ses aspects, l'invention propose un moule pour la fabrication de récipients, notamment de bouteilles, à partir d'ébauches en matériau thermoplastique, notamment en PET, lequel moule, étant agencé conformément à l'invention pour la mise en oeuvre du procédé de mesure mentionné ci-dessus, comporte au moins un capteur capacitif inséré dans sa paroi définissant la cavité de moulage, la face frontale du capteur affleurant la surface de la cavité et étant conformée dans la continuité de forme de ladite surface.

Dans un mode de réalisation préféré, le capteur comporte une face frontale sur laquelle apparaissent une électrode centrale et une électrode externe séparées par une région intermédiaire annulaire en matériau diélectrique, ladite électrode externe étant en contact électriquement conducteur avec le moule formant masse électrique. Compte tenu de la faiblesse des signaux de sortie du capteur difficiles à détecter dans un environnement électromagnétique peu favorable, on peut avantageusement prévoir que le capteur comporte en outre une électrode intermédiaire (électrode de garde) interposée entre l'électrode centrale et l'électrode externe ; avantageusement, cette électrode intermédiaire (électrode de garde) est connectée de façon à être à un potentiel différent des électrodes centrale et externe entre lesquelles elle est interposée.

Pour obtenir un rendement optimum du capteur capacitif, il est souhaitable qu'il soit logé et fixé dans un alésage de la paroi du moule qui s'étend approximativement perpendiculairement à la surface de la cavité de moulage lorsque cette surface est plane ou au plan tangent à la surface de la cavité de moulage lorsque cette surface est courbe.

Dans un mode de mise en oeuvre avantageux permettant un contrôle fiable de l'épaisseur de la paroi du récipient en divers emplacements sélectionnés de celle-ci, le moule comporte plusieurs capteurs capacitifs disposés en divers emplacements respectifs de la paroi définissant la cavité de moulage.

Les dispositions conformes à l'invention trouvent une application particulièrement intéressante, bien que non exclusive, dans les moules du type moule portefeuille comportant au moins deux demi-moules écartables l'un de l'autre par rotation autour d'un axe de rotation autour d'un axe commun, qui sont couramment utilisés dans les machines de la Demanderesse.

Dans ce cas, il est possible de faire en sorte que chaque demi-moule soit pourvu d'au moins un capteur capacitif, autrement dit de prévoir plusieurs capteurs capacitifs répartis dans les deux demi-moules, ou bien au contraire qu'un seul demi-moule soit pourvu d'au moins un capteur capacitif, autrement dit que le ou les capteurs capacitifs soient tous regroupés dans un seul des demi-moules de manière à simplifier la conception du moule. On peut également prévoir que le moule comporte en outre un fond de moule distinct des demi-moules et que le fond de moule soit pourvu d'au moins un capteur capacitif.

On peut en outre prévoir que le moule soit agencé de manière que les fils de connexion du capteur capacitif soient engagés dans une saignée creusée dans la face externe du moule et sortent du moule à la partie inférieure ou à la partie supérieure de celui-ci. Ce mode de mise en oeuvre se révèle particulièrement intéressant lorsque le moule est constitué par assemblage de plusieurs constituants, notamment lorsque l'empreinte de moulage est évidée dans des coquilles (dans lesquelles sont insérés les capteurs) assemblées à des porte-coquilles eux-mêmes supportés dans des porte-moules ou lorsque les demi-moules sont supportés dans des porte-moules, afin de ne pas affaiblir mécaniquement respectivement les porte-coquilles et/ou les porte-moules par un ou plusieurs perçages pour le passage des fils de connexion.

Les distributeurs de produits conditionnés dans des récipients, notamment des bouteilles, en matière thermoplastique souhaitent vivement disposer de récipients dont la surface externe soit lisse et d'un aspect impeccable, sans aucune aspérité ou trace visible. Il est donc nécessaire que la présence d'un ou de plusieurs capteurs capacitifs dans le moule n'entraîne aucun défaut de surface sur le récipient final. Pour ce faire, selon un troisième de ses aspects, l'invention propose un procédé de fabrication d'un moule tel qu'exposé plus haut, lequel procédé se caractérise en ce qu'il comprend les étapes suivantes :
- on usine dans le moule une ébauche de la cavité de moulage,
- on met en place un capteur capacitif dans un alésage pratiqué dans la paroi du moule sensiblement transversalement à la surface définitive de la cavité de moulage et on l'y fixe dans sa position de montage de manière que la face frontale du capteur fasse saillie au-delà, vers l'intérieur, de la surface définitive de la cavité de moulage, et
- on termine l'usinage de la cavité de moulage en usinant simultanément le capteur capacitif de manière que la face frontale de celui-ci soit en affleurement de la surface de la cavité de moulage et conformé dans la continuité de forme de ladite surface.

Grâce à ce processus de fabrication du moule, on parvient à intégrer la face frontale du capteur capacitif dans une parfaite continuité de conformation avec la surface de la cavité de moulage, de telle sorte qu'aucune trace significative n'apparaît sur le récipient final. Bien entendu, la mise en oeuvre du procédé qui vient d'être exposé implique que la partie terminale du capteur avoisinant sa face frontale soit suffisamment dure pour pouvoir être usinée de façon homogène avec la surface de la cavité de moulage.

Enfin, selon un quatrième de ses aspects, l'invention propose une installation de moulage pour la fabrication de récipients, notamment de bouteilles, par soufflage ou étirage-soufflage d'ébauches en matière thermoplastique, notamment en PET, dans un moule comportant au moins une cavité de moulage, laquelle installation, étant agencée conformément à l'invention pour la mise en oeuvre du procédé de mesure mentionné plus haut, se caractérise en ce qu'elle comporte :
- au moins un moule agencé conformément à l'invention comme indiqué plus haut et comportant au moins un capteur capacitif inséré dans la paroi dudit moule définissant une cavité de moulage, la face frontale du capteur affleurant la surface de la cavité et étant conformée dans la continuité de forme de ladite surface, et
- des moyens de traitement pour traiter le signal du capteur et en déduire une information représentative d'une caractéristique du récipient.

La détection mise en oeuvre dans le cadre de l'invention est fondée sur la perturbation du champ électrostatique sur la face frontale du capteur lorsque cette face frontale est mise en présence d'un corps isolant (le matériau de la paroi du récipient) à coefficient diélectrique différent de celui de l'air (absence de paroi de récipient à son contact), laquelle perturbation est représentative de la caractéristique à déterminer.

Grâce à ces dispositions, la détection est conduite sur chaque récipient venant d'être moulé, au moment où, sous l'action du soufflage pneumatique sous pression, sa paroi est plaquée contre la surface de la cavité de moulage. Cette détection ne nécessite aucune manoeuvre particulière ni aucun mécanisme spécifique et se produit automatiquement lors de la modification du champ électrostatique à la surface du capteur en présence de la paroi du récipient.

Avantageusement, l'installation peut comprendre des moyens d'affichage de la valeur détectée de la caractéristique du récipient en regard du capteur de manière à alerter un opérateur surveillant le fonctionnement de l'installation.

Toutefois, il est préférable de prévoir, éventuellement en combinaison avec la disposition précédente, que l'installation comprenne des moyens de mémorisation de la valeur détectée de la caractéristique du récipient en regard du capteur ; alors, de façon avantageuse, les moyens de mémorisation peuvent être propres à mémoriser un nombre prédéterminé de valeurs détectées par le capteur capacitif pour des récipients fabriqués successivement et il est prévu des moyens d'analyse de ce nombre prédéterminé de valeurs détectées qui sont propres à déterminer une variation durable des valeurs détectées représentative d'une variation durable de la caractéristique des récipients fabriqués successivement : un tel agencement permet de s'affranchir de variations unitaires ou de très brève durée et de ne retenir que les variations affirmées dues à une dérive des conditions de fonctionnement de l'installation susceptibles d'affecter un grand nombre de récipients.

En particulier alors, on peut faire en sorte que les moyens d'analyse soient raccordés à un automate gérant les paramètres de fonctionnement du moule de manière que lesdits paramètres de fonctionnement soient modifiés en fonction de la variation détectée de la caractéristique. On automatise ainsi le fonctionnement de l'installation, et les corrections appropriées sont apportées sans qu'il soit besoin d'arrêter l'installation, ni même sans intervention d'un opérateur.

De façon intéressante, on peut faire en sorte que l'installation soit équipée d'au moins un moule pourvu de plusieurs capteurs capacitifs disposés en divers emplacements respectifs de la paroi définissant la cavité de moulage. Il est alors possible de contrôler la caractéristique en divers emplacements de chaque récipient, par exemple sur l'épaule ou sur le corps au voisinage de l'épaule, sur le corps (éventuellement en plusieurs emplacements écartés les uns des autres), sur le fond ou sur le corps au voisinage du fond.

Les dispositions de l'invention peuvent trouver une application particulièrement intéressante dans une installation de moulage rotative du type carrousel tournant comportant plusieurs moules disposés périphériquement : dans ce cas, il est souhaitable qu'au moins un moule soit muni d'au moins un capteur capacitif.

La présence d'au moins un moule équipé selon l'invention permet d'effectuer un échantillonnage en vue d'actions correctives ; lorsque tous les moules sont ainsi équipés, il devient possible, outre les actions correctives, de séparer le cas échéant les récipients conformes des récipients non conformes.

Dans une application préférée des dispositions de l'invention, la caractéristique mesurée à l'aide d'au moins un capteur capacitif est l'épaisseur de la paroi du récipient en regard du capteur capacitif.

L'invention sera mieux comprise à la lecture de la description détaillée qui suit de certains modes de réalisation préférés donnés à titre purement illustratif. Dans cette description, on se réfère aux dessins annexés sur lesquels :
- la figure 1A est une vue schématique de côté en coupe d'une partie d'un moule illustrant les dispositions conformes à l'invention ;
- la figure 1B est une vue schématique en coupe selon la ligne IB-IB de la figure 1A ;
- la figure 2 est une vue schématique analogue à celle de la figure 1B, montrant une variante de réalisation intéressante ;
- la figure 3 est une vue en perspective d'un demi-moule montrant la cavité de moulage équipée de capteurs capacitifs conformément à l'invention ;
- les figures 4A à 4D sont des représentations schématiques illustrant des étapes d'un- procédé de fabrication d'un moule agencé conformément à l'invention ;
- la figure 5 est un schéma illustrant la constitution d'une installation de fabrication de moulage de récipients agencée conformément à l'invention ; et
- la figure 6 est une représentation schématique d'un mode de réalisation préféré d'une installation de fabrication de récipients agencée selon l'invention.

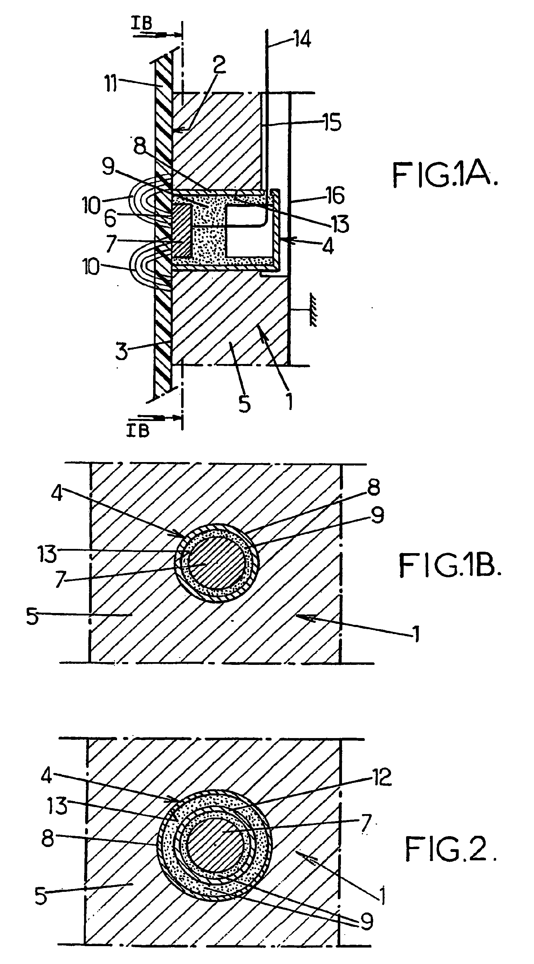

En se reportant tout d'abord aux figures 1A et 1B, il y est représenté une partie de moule 1 avec une cavité 2 de moulage, définie par une surface 3 de moulage, pour la fabrication de récipients, notamment de bouteilles, à partir d'ébauches (préformes ou récipients intermédiaires) en matériau thermoplastique tel que le PET.

Comme cela est bien visible à la figure 1A, il est prévu, conformément à l'invention, au moins un capteur capacitif 4 inséré dans la paroi 5 du moule bordant la cavité 2 de moulage, ce capteur capacitif 4 ayant une face frontale 6 qui affleure la surface 3 de moulage de la cavité et qui est conformée dans la continuité de forme de ladite surface 3.

Sur la face frontale 6 du capteur capacitif 4 apparaissent une électrode 7 centrale et une électrode 8 externe qui sont séparées par une région 9 intermédiaire annulaire en matériau diélectrique, ladite électrode 8 externe étant en contact électriquement conducteur avec le moule 1 formant masse électrique, comme visible à la figure 1A ; en pratique, comme montré à la figure 1B, l'électrode 8 externe se présente sous forme d'un boîtier métallique qui entoure une masse de matériau diélectrique dans laquelle est noyée l'électrode 7 centrale et qui est mis directement en contact avec le moule.

Lorsque l'électrode 7 centrale est sous tension, le champ électrostatique, schématisé en 10, qui s'établit entre les électrodes 7 et 8 à la face frontale 6 du capteur est perturbé par l'intrusion d'un corps en matériau isolant telle que la paroi 11 d'un récipient qui vient d'être soufflé et qui s'applique alors contre la surface 3 de moulage de la cavité 2 de moulage et la face frontale 6 du capteur capacitif 4. La perturbation induite par la présence de la paroi 11 est dépendante d'une caractéristique physique de cette paroi 11. Le signal de sortie du capteur capacitif 4 véhicule donc une information représentative de cette caractéristique, qui peut être notamment la densité du matériau constitutif de la paroi en regard du capteur (laquelle densité est représentative du taux de cristallinité du matériau) ou bien aussi l'épaisseur de la paroi en regard du capteur. Dans la suite de la description, on se référera plus particulièrement à la détermination de cette épaisseur, étant entendu que les dispositions de l'invention ne se limitent pas à cette seule application.

Le signal de sortie du capteur capacitif 4 étant généralement très faible, il est intéressant, dans une variante de réalisation, d'agencer le capteur capacitif 4 comme illustré à la figure 2 avec en outre une électrode 12 intermédiaire (ou électrode de garde) interposée entre l'électrode 7 centrale et l'électrode 8 externe.

Comme on peut le voir clairement à la figure 1A, pour que le capteur capacitif 4 soit mis en oeuvre dans les conditions de performances optimales, le capteur capacitif 4 est logé et fixé dans un alésage 13 de la paroi 5 du moule 1 qui s'étend approximativement perpendiculairement à la surface 3 de moulage de la cavité 2 de moulage lorsque cette surface est plane (cas illustré à la figure 1A) ou au plan tangent à la surface 3 de moulage de la cavité 2 de moulage lorsque cette surface est courbe.

Il est souhaitable, comme montré à la figure 1A, que les fils 14 de connexion du capteur capacitif 4 soient engagés dans une saignée 15 creusée dans la face 16 externe de la paroi 5 du moule et sortent du moule à la partie inférieure ou à la partie supérieure de celui-ci. Ainsi, les dispositions conformes à l'invention peuvent être mises en oeuvre même dans le cas de moules à structure composite, par exemple dans le cas de moules formés de deux coquilles de moulage fixées à deux porte-coquilles respectifs, eux-mêmes pouvant être supportés par deux porte-moules respectifs sans qu'il soit besoin de percer le porte-coquille, et le porte-moule éventuel : on écarte ainsi le risque d'affaiblissement que pourrait apporter l'alésage dans le porte-coquille et éventuellement le porte-moule.

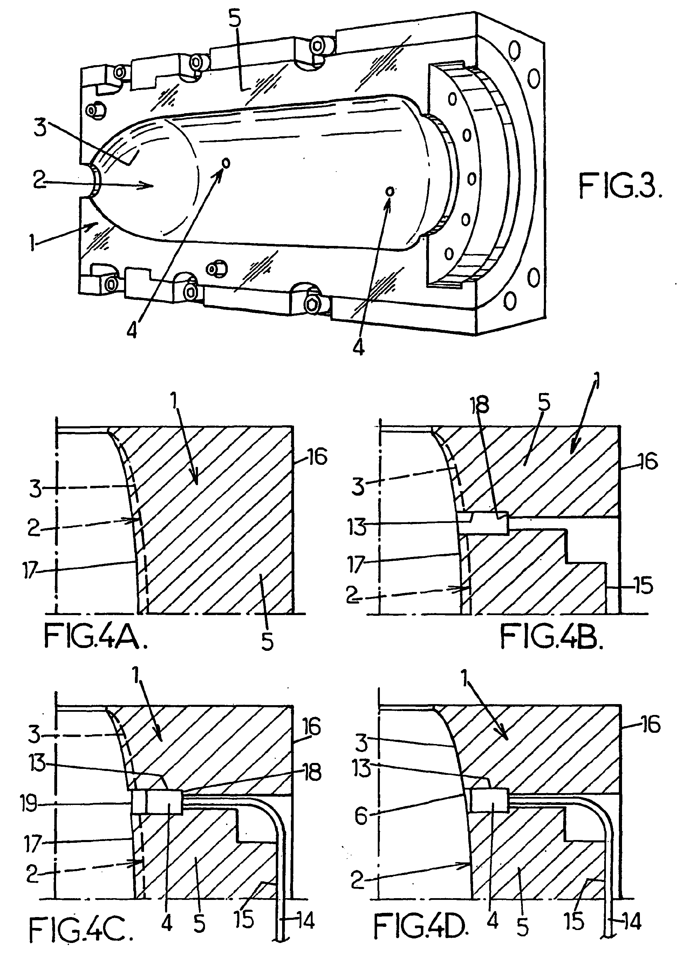

Dans la pratique, on pourra installer dans un moule plusieurs capteurs capacitifs 4 disposés en divers emplacements respectifs de la paroi 5 définissant la cavité 2 de moulage, comme montré à la figure 3. En particulier, il peut être intéressant de disposer des capteurs dans certains emplacements privilégiés de la cavité 2 de moulage, tels que dans l'épaule ou le corps au voisinage immédiat de l'épaule, dans le corps en un ou plusieurs emplacements (cas illustré dans l'exemple de la figure 3 sur laquelle est montré un demi-moule), dans le fond ou le corps au voisinage immédiat du fond.

Les dispositions conformes à l'invention peuvent trouver application dans tout type de moule. Cependant, elles doivent trouver tout particulièrement application dans les moules du type portefeuille qui sont aujourd'hui largement répandus et qui comportent au moins deux demi-moules écartables l'un de l'autre par rotation autour d'un axe de rotation autour d'un axe commun (c'est un demi-moule pour ce type de moule portefeuille qui est montré à la figure 3). Dans ce cas, on peut prévoir que chaque demi-moule soit pourvu d'au moins un capteur capacitif, ou bien encore qu'un seul demi-moule soit pourvu d'au moins un capteur capacitif de manière notamment à simplifier le câblage.

Il est particulièrement important de veiller à ce que la présence du capteur capacitif 4 affleurant la surface 3 de moulage de la cavité 2 de moulage n'entraîne aucune marque sensiblement visible sur la surface externe de la paroi du récipient final, et à cette fin il est nécessaire de veiller à ce que la face frontale du capteur capacitif 4 soit parfaitement affleurante et conformée dans la parfaite continuité de forme de la surface 3 de moulage de la cavité 2 de moulage. Aussi l'invention prévoit-elle un procédé pour aboutir à cet affleurement parfait, procédé qui est exposé ci-après en référence aux figures 4A à 4D qui montrent une partie d'un moule en coupe diamétrale.

On commence, comme montré à la figure 4A, par usiner, dans la paroi 5 du moule 1, une ébauche 17 de la cavité de moulage selon toute technique appropriée (la surface 3 de moulage définitive de la cavité 2 de moulage est dessinée en tirets).

Puis, comme montré à la figure 4B on peut à ce stade (si cette étape n'a pas été réalisée au préalable, par exemple avant l'usinage de l'ébauche de la cavité) usiner un alésage 13 à travers la paroi 5 du moule, lequel alésage 13 s'étend sensiblement radialement à la surface 3 de moulage définitive de la cavité 2 de moulage ; de préférence, l'alésage 13 comporte un épaulement 18 axial.

Ensuite on met en place un capteur -capacitif 4 dans cet alésage 13, en butée axiale contre l'épaulement 18, et on l'y fixe dans sa position de montage de manière que la face frontale 19 du capteur capacitif 4 fasse saillie au-delà, vers l'intérieur, de la surface 3 de moulage définitive de la cavité 2 de moulage, comme cela apparaît à la figure 4C.

Enfin, on termine l'usinage de la cavité 2 de moulage en usinant simultanément le capteur capacitif 4 de manière que la face frontale 6 de celui-ci soit en affleurement de la surface 3 de moulage de la cavité 2 de moulage et conformée dans la continuité de forme de ladite surface 3, comme cela est montré à la figure 4D.

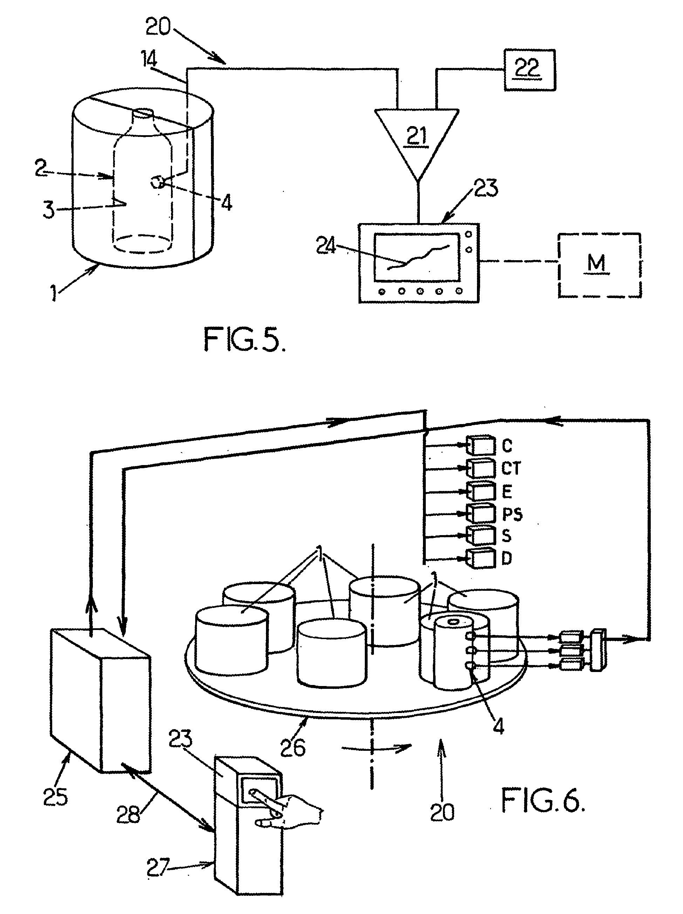

A la figure 5 est illustrée schématiquement une installation de moulage, désignée dans son ensemble par la référence numérique 20, pour la fabrication de récipients, notamment de bouteilles, par soufflage ou étirage-soufflage d'ébauches en matière thermoplastique, notamment en PET, dans un moule 1 comportant une cavité 2 de moulage, installation de moulage qui est équipée d'au moins un capteur capacitif 4 dans les conditions indiquées plus haut, c'est-à-dire que le capteur capacitif 4 est inséré dans la paroi 5 du moule 1, la face frontale 6 du capteur affleurant la surface 3 de moulage de la cavité 2 de moulage et étant conformée dans la continuité de forme de ladite surface 3. Des moyens 23 de traitement de signal traitent le signal de sortie du capteur capacitif 4 et délivrent une information représentative de la valeur détectée de l'épaisseur de la paroi 11 du récipient en regard du capteur capacitif 4.

On peut, comme illustré à la figure 5, faire intervenir des moyens 21 de comparaison qui reçoivent le signal de sortie du capteur capacitif 4 et comparent sa valeur avec une valeur de référence tenue dans une mémoire 22, le signal de comparaison étant alors délivré auxdits moyens 23 de traitement de signal.

Les moyens 23 de traitement du signal de sortie des moyens 21 de comparaison peuvent notamment comprendre, comme montré à la figure 5, des moyens 24 d'affichage de la valeur détectée de l'épaisseur de la paroi 11 du récipient en regard du capteur capacitif 4, et/ou ils peuvent comprendre des moyens (M) de mémorisation de la valeur détectée de l'épaisseur de la paroi 11 du récipient. En particulier, on peut alors prévoir que les moyens (M) de mémorisation soient propres à mémoriser un nombre prédéterminé de valeurs détectées par le capteur capacitif pour des récipients fabriqués successivement et en ce qu'il soit prévu des moyens d'analyse de ce nombre prédéterminé de valeurs détectées qui soient propres à déterminer une variation durable des valeurs détectées représentative d'une variation durable de l'épaisseur dans les récipients fabriqués successivement : ce processus de traitement permet de s'affranchir de variations ponctuelles n'affectant qu'un seul récipient ou qu'un petit nombre de récipients sans effet durable.

Dans ce cas, on peut prévoir avantageusement, comme montré à la figure 6, qui illustre d'une autre manière schématique une installation de moulage, désignée dans son ensemble par la référence numérique 20, que les moyens 23 de traitement de signal et/ou les moyens d'analyse précités soient raccordés à un automate 25 gérant les paramètres de fonctionnement du moule 1 de manière que lesdits paramètres de fonctionnement soient modifiés en fonction de la variation durable détectée (par paramètres de fonctionnement, on peut comprendre sans que ceci soit limitatif ou systématique par exemple les moyens C de compensation de pression associés au moule, la commande de tuyère de soufflage CT, la commande de la tige d'étirage E dans le cas, fréquent, où le moulage s'effectue par un processus d'étirage-soufflage, la commande de présoufflage PS, la commande de soufflage S, la commande de dégazage ou échappement D, les paramètres de chauffe des préformes, etc.).

Il peut, comme évoqué plus haut, être prévu plusieurs capteurs capacitifs disposés en divers emplacements respectifs de la paroi 5 définissant la cavité 2 de moulage dans un moule 1. Dans ce cas, l'automate prend en compte de façon sélective toutes les informations issues de ces capteurs capacitifs respectifs et gère le fonctionnement du moule de façon sélective dans les diverses parties de celui-ci.

Les dispositions conformes à l'invention trouvent une application préférée, bien que non exclusive, dans le cas d'une installation 20 de moulage rotative du type carrousel tournant 26, illustrée à la figure 6, et comportant plusieurs moules 1 disposés périphériquement, installation qui est alors agencée comme indiqué plus haut avec au moins un moule 1 muni d'au moins un capteur capacitif 4 (trois capteurs sont représentés à la figure 6). Selon la capacité de traitement des informations et/ou selon la complexité acceptable pour l'installation (complexité dont est tributaire son coût), seul un moule 1 peut être équipé d'un capteur capacitif ou d'un ensemble de capteurs capacitifs (on suppose alors que le fonctionnement de ce moule, qui est donc utilisé pour un échantillonnage, est représentatif du fonctionnement de l'ensemble des moules de l'installation), ou bien chaque moule peut être équipé d'un capteur ou d'un ensemble de capteurs capacitifs (le fonctionnement de chaque moule étant alors géré de façon isolée). Dans l'exemple illustré à la figure 6, l'automate 25 est représenté relié par un bus 28 de transmission de données à un poste de commande centralisée (PCC) 27 qui peut alors inclure par exemple les moyens 21 de comparaison et mémoire 22 contenant la valeur de référence évoqués plus haut, ainsi que les moyens 23 de traitement de signal et les moyens 24 d'affichage.

## Revendications

1. Procédé de mesure d'au moins une caractéristique physique de récipients, notamment de bouteilles, fabriqués par soufflage ou étirage-soufflage d'ébauches en matière thermoplastique, notamment en PET, dans un moule comportant au moins une cavité de moulage, **caractérisé en ce que** ladite mesure est faite par détection d'un effet capacitif ou d'une variation d'effet capacitif dans le moule au cours du soufflage ou de l'étirage-soufflage.

2. Procédé selon la revendication 1, **caractérisé en ce que** la caractéristique mesurée est l'épaisseur de la paroi du récipient en au moins un emplacement de celui-ci.

3. Procédé selon la revendication 1, **caractérisé en ce que** la caractéristique mesurée est la densité du matériau de la paroi du récipient en au moins un emplacement de celui-ci.

4. Procédé selon l'une quelconque des revendications 1 à 3, **caractérisé en ce que** la mesure est faite entre la fin du soufflage ou étirage-soufflage et le début de l'ouverture du moule, alors que la paroi du récipient est en contact étroit avec la surface de moulage de la cavité de moulage.

5. Moule (1) pour la fabrication de récipients, notamment de bouteillers, à partir d'ébauches en matériau thermoplastique, notamment en PET, **caractérisé en ce que**, pour la mise en oeuvre du procédé de mesure selon l'une quelconque des revendications 1 à 4, ledit moule comporte au moins un capteur capacitif (4) inséré dans sa paroi (5) définissant la cavité (2) de moulage, la face frontale (6) du capteur capacitif (4) affleurant la surface (3) de moulage de la cavité (2) de moulage et étant conformée dans la continuité de forme de ladite surface (3).

6. Moule selon la revendication 5, **caractérisé en ce que** le capteur capacitif (4) comporte une face frontale (6) sur laquelle apparaissent une électrode (7) centrale et une électrode (8) externe séparées par une région (9) intermédiaire annulaire en matériau diélectrique, ladite électrode (8) externe étant en contact électriquement conducteur avec le moule (1) formant masse électrique.

7. Moule selon la revendication 5 ou 6, **caractérisé en ce que** le capteur capacitif (4) comporte en outre une électrode (12) intermédiaire (électrode de garde) interposée entre l'électrode (7) centrale et l'électrode (8) externe.

8. Moule selon la revendication 7, **caractérisé en ce que** l'électrode (12) intermédiaire (électrode de garde) est connectée de façon à être à un potentiel différent des électrodes (7) centrale et (8) externe entre lesquelles elle est interposée.

9. Moule selon l'une quelconque des revendications 5 à 8, **caractérisé en ce que** le capteur capacitif (4) est logé et fixé dans un alésage (13) de la paroi (5) du moule (1) qui s'étend approximativement perpendiculairement à la surface (3) de moulage de la cavité (2) de moulage lorsque cette surface est plane ou au plan tangent à la surface (3) de moulage de la cavité de moulage lorsque cette surface est courbe.

10. Moule selon l'une quelconque des revendications 5 à 9, **caractérisé en ce qu'**il comporte plusieurs capteurs capacitifs (4) disposés en divers emplacements respectifs de la paroi (5) définissant la cavité (2) de moulage.

11. Moule selon l'une quelconque des revendications 5 à 10, **caractérisé en ce qu'**il est du type moule portefeuille comportant au moins deux demi-moules écartables l'un de l'autre par rotation autour d'un axe de rotation autour d'un axe commun.

12. Moule selon la revendication 11, **caractérisé en ce que** chaque demi-moule est pourvu d'au moins un capteur capacitif (4).

13. Moule selon la revendication 11, **caractérisé en ce qu'**un seul demi-moule est pourvu d'au moins un capteur capacitif (4).

14. Moule selon la revendication 11, **caractérisé en ce qu'**il comporte en outre un fond de moule distinct des demi-moules et **en ce que** le fond de moule est pourvu d'au moins un capteur capacitif (4).

15. Moule selon l'une quelconque des revendications 5 à 14, **caractérisé en ce que** les fils (14) de connexion du capteur capacitif (4) sont engagés dans une saignée (15) creusée dans la face (16) externe du moule et sortent du moule (1) à la partie inférieure ou à la partie supérieure de celui-ci.

16. Procédé de fabrication d'un moule selon l'une quelconque des revendications 5 à 15, **caractérisé en ce qu'**il comprend les étapes suivantes :
- on usine dans le moule (1) une ébauche (17) de la cavité (2) de moulage,
- on met en place un capteur capacitif (4) dans un alésage (13) pratiqué dans la paroi (5) du moule sensiblement transversalement à la surface (3) de moulage définitive de la cavité (2) de moulage et on l'y fixe dans sa position de montage de manière que la face frontale (19) du capteur capacitif (4) fasse saillie au-delà, vers l'intérieur, de la surface (3) de moulage définitive de la cavité (2) de moulage, et
- on termine l'usinage de la cavité (2) de moulage en usinant simultanément le capteur capacitif (4) de manière que la face frontale (6) terminale de celui-ci soit en affleurement de la surface (3) de moulage de la cavité (2) de moulage et conformée dans la continuité de forme de ladite surface (3).

17. Installation de moulage pour la fabrication de récipients, notamment de bouteilles, par soufflage ou étirage-soufflage d'ébauches en matière thermoplastique, notamment en PET, dans un moule (1) comportant au moins une cavité (2) de moulage,
**caractérisée en ce que**, pour la mise en oeuvre du procédé de mesure selon l'une quelconque des revendications 1 à 4, ladite installation comporte :
- au moins un moule agencé selon l'une quelconque des revendications 5 à 15 et comportant un capteur capacitif (4) inséré dans la paroi (5) dudit moule définissant la cavité (2) de moulage, la face frontale (6) du capteur capacitif (4) affleurant la surface (3) de moulage de la cavité (2) de moulage et étant conformée dans la continuité de forme de ladite surface (3), et
- des moyens (23) de traitement pour traiter le signal du capteur capacitif (4) et en déduire une information représentative d'une caractéristique du récipient.

18. Installation selon la revendication 17, **caractérisée en ce qu'**elle comprend des moyens (24) d'affichage de la valeur détectée de la caractéristique du récipient en regard du capteur capacitif (4).

19. Installation selon la revendication 17 ou 18, **caractérisée en ce qu'**elle comprend des moyens (M) de mémorisation de la valeur détectée de la caractéristique du récipient en regard du capteur capacitif (4).

20. Installation selon la revendication 19, **caractérisée en ce que** les moyens (M) de mémorisation sont propres à mémoriser un nombre prédéterminé de valeurs détectées par le capteur capacitif (4) pour des récipients fabriqués successivement et **en ce qu'**il est prévu des moyens d'analyse de ce nombre prédéterminé de valeurs détectées qui sont propres à déterminer une variation durable des valeurs détectées représentative d'une variation durable de la caractéristique des récipients fabriqués successivement.

21. Installation selon la revendication 20, **caractérisée en ce que** les moyens d'analyse sont raccordés à un automate (25) gérant les paramètres de fonctionnement de l'installation de manière que lesdits paramètres de fonctionnement soient modifiés en fonction de la variation détectée de la caractéristique.

22. Installation selon l'une quelconque des revendications 17 à 21, **caractérisée en ce que** plusieurs capteurs capacitifs (4) sont disposés en divers emplacements respectifs de la paroi (5) définissant la cavité (2) de moulage.

23. Installation de moulage rotative du type carrousel tournant (26) comportant plusieurs moules disposés périphériquement, **caractérisée en ce qu'**elle est agencée selon l'une quelconque des revendications 17 à 22, au moins un moule (1) étant muni d'au moins un capteur capacitif (4).

24. Installation selon l'une quelconque des revendications 17 à 23, **caractérisée en ce que** la caractéristique mesurée à l'aide d'au moins un capteur capacitif est l'épaisseur de la paroi du récipient en regard du capteur capacitif.

## Claims

1. A method for measuring at least one physical characteristic of containers, particularly of bottles, manufactured by blow-molding or stretch-blow-molding of parisons made of thermoplastic, particularly of PET, in a mold comprising at least one molding cavity,
**characterized in that** said measurement is made by detecting a capacitive effect or a variation in capacitive effect in the mold during blow-molding or stretch-blow-molding.

2. The method as claimed in claim 1, **characterized in that** the measured characteristic is the thickness of the wall of the container at least at one location thereon.

3. The method as claimed in claim 1, **characterized in that** the measured characteristic is the density of the material of the wall of the container at least at one location thereon.

4. The method as claimed in any one of claims 1 to 3, **characterized in that** the measurement is made between the end of blow-molding or stretch-blow-molding and the start of mold opening while the wall of the container is in close contact with the molding surface of the molding cavity.

5. A mold (1) for the manufacture of containers, particularly bottles, from parisons made of thermoplastic, particularly of PET, **characterized in that**, in order to implement the measurement method as claimed in any one of claims 1 to 4, said mold comprises at least one capacitive sensor (4) inserted in its wall (5) defining the molding cavity (2), the front face (6) of the capacitive sensor (4) lying flush with the molding surface (3) of the molding cavity (2) and being shaped as a continuation of the shape of said surface (3).

6. The mold as claimed in claim 5, **characterized in that** the capacitive sensor (4) comprises a front face (6) on which there are a central electrode (7) and an external electrode (8) which are separated by an annular intermediate region (9) of dielectric material, said external electrode (8) being in electrically conducting contact with the mold (1) which forms the electrical ground.

7. The mold as claimed in claim 5 or 6, **characterized in that** the capacitive sensor (4) further comprises an intermediate electrode (12) (field electrode) interposed between the central electrode (7) and the external electrode (8).

8. The mold as claimed in claim 7, **characterized in that** the intermediate electrode (12) (field electrode) is connected in such a way as to be at a different potential than the central (7) and external (8) electrodes between which it is interposed.

9. The mold as claimed in any one of claims 5 to 8, **characterized in that** the capacitive sensor (4) is housed and fixed in a bore (13) in the wall (5) of the mold (1) which runs approximately perpendicular to the molding surface (3) of the molding cavity (2) when this surface is flat or to the plane tangential to the molding surface (3) of the molding cavity when this surface is curved.

10. The mold as claimed in any one of claims 5 to 9, **characterized in that** it comprises several capacitive sensors (4) positioned at various respective locations in the wall (5) defining the molding cavity (2).

11. The mold as claimed in any one of claims 5 to 10, **characterized in that** it is of the hinged mold type comprising at least two half-molds that can be parted from one another by rotation about an axis of rotation about a common axis.

12. The mold as claimed in claim 11, **characterized in that** each half-mold is provided with at least one capacitive sensor (4).

13. The mold as claimed in claim 11, **characterized in that** only one half-mold is provided with at least one capacitive sensor (4).

14. The mold as claimed in claim 11, **characterized in that** it further comprises a mold bottom distinct from the half-molds and **in that** the mold bottom is provided with at least one capacitive sensor (4).

15. The mold as claimed in any one of claims 5 to 14, **characterized in that** the wires (14) connecting the capacitive sensor (4) are engaged in a cut (15) made in the external face (16) of the mold and leave the mold (1) at the lower part or at the upper part thereof.

16. A method of manufacturing a mold as claimed in any one of claims 5 to 15, **characterized in that** it comprises the following steps:
- a rough form (17) of the molding cavity (2) is machined in the mold (1),
- a capacitive sensor (4) is placed in a bore (13) made in the wall (5) of the mold substantially transversely with respect to the final molding surface (3) of the molding cavity (2) and is secured therein in its mounted position in such a way that the front face (19) of the capacitive sensor (4) protrudes inward beyond the final molding surface (3) of the molding cavity (2), and
- the machining of the molding cavity (2) is completed, at the same time machining the capacitive sensor (4) in such a way that the terminal front face (6) thereof lies flush with the molding surface (3) of the molding cavity (2) and is shaped as a continuation of the shape of said surface (3).

17. A molding installation for manufacturing containers, particularly bottles, by blow-molding or stretch-blow-molding parisons made of thermoplastic, particularly of PET, in a mold (1) comprising at least one molding cavity (2),
**characterized in that**, in order to implement the measurement method as claimed in any one of claims 1 to 4, said installation comprises:
- at least one mold arranged as claimed in any one of claims 5 to 15 and provided with a capacitive sensor (4) inserted in the wall (5) of said mold defining the molding cavity (2), the front face (6) of the capacitive sensor (4) lying flush with the molding surface (3) of the molding cavity (2) and being shaped as a continuation of the shape of said surface (3), and
- processing means (23) for processing the signal from the capacitive sensor (4) and deducing therefrom information representative of a characteristic of the container.

18. The installation as claimed in claim 17, **characterized in that** it comprises means (24) for displaying the detected value of the characteristic of the container facing the capacitive sensor (4).

19. The installation as claimed in claim 17 or 18, **characterized in that** it comprises means (M) for storing the detected value of the characteristic of the container facing the capacitive sensor (4).

20. The installation as claimed in claim 19, **characterized in that** the storage means (M) are adapted to store a predetermined number of values detected by the capacitive sensor (4) in respect of containers manufactured in succession and **in that** means for analyzing this predetermined number of detected values are provided and are adapted to determine a variation over time of the detected values representative of a variation over time of the characteristic of the containers manufactured in succession.

21. The installation as claimed in claim 20, **characterized in that** the analysis means are connected to an automatic controller (25) managing the parameters of operation of the installation in such a way that said operating parameters are modified on the basis of the detected variation in the characteristic.

22. The installation as claimed in any one of claims 17 to 21, **characterized in that** several capacitive sensors (4) are positioned at various respective locations on the wall (5) defining the molding cavity (2).

23. A rotary molding installation of the rotary carousel type (26) comprising several molds arranged along the periphery, **characterized in that** it is arranged as claimed in any one of claims 17 to 22, at least one mold (1) being provided with at least one capacitive sensor (4).

24. The installation as claimed in any one of claims 17 to 23, **characterized in that** the characteristic measured using at least one capacitive sensor is the wall thickness of the container facing the capacitive sensor.

## Patentansprüche

1. Verfahren zum Messen mindestens eines physikalischen Merkmals von Behältern, insbesondere von Flaschen, die durch Blasen oder Streckblasen von Vorformlingen aus Thermoplast, insbesondere aus PET, in einer mindestens einen Formhohlraum aufweisenden Form hergestellt wurden, **dadurch gekennzeichnet, dass** die Messung durch Erfassung eines kapazitiven Effekts oder einer Änderung eines kapazitiven Effekts in der Form während des Blasens oder des Streckblasens vorgenommen wird.

2. Verfahren nach Anspruch 1, **dadurch gekennzeichnet, dass** das gemessene Merkmal die Dicke der Wand des Behälters an mindestens einer Stelle von diesem ist.

3. Verfahren nach Anspruch 1, **dadurch gekennzeichnet, dass** das gemessene Merkmal die Dichte des Werkstoffs der Wand des Behälters an mindestens einer Stelle von diesem ist.

4. Verfahren nach einem der Ansprüche 1 bis 3, **dadurch gekennzeichnet, dass** die Messung zwischen dem Ende des Blasens oder Streckblasens und dem Anfang der Öffnung der Form vorgenommen wird, während die Wand des Behälters mit der Formoberfläche des Formhohlraums in engem Kontakt ist.

5. Form (1) für die Herstellung von Behältern, insbesondere von Flaschen, aus Vorformlingen aus Thermoplast, insbesondere aus PET, **dadurch gekennzeichnet, dass** diese Form für die Durchführung des Messverfahrens nach einem der Ansprüche 1 bis 4 mindestens einen kapazitiven Fühler (4) umfasst, der in ihre den Formhohlraum (2) begrenzende Wand (5) eingeführt ist, wobei die Frontseite (6) des kapazitiven Fühlers (4) mit der Formoberfläche (3) des Formhohlraums (2) bündig ist und in der Formkontinuität dieser Oberfläche (3) ausgeformt ist.

6. Form nach Anspruch 5, **dadurch gekennzeichnet, dass** der kapazitive Fühler (4) eine Frontseite (6) umfasst, an der eine Mittelelektrode (7) und eine Außenelektrode (8) erscheint, die durch einen ringförmigen Zwischenbereich (9) aus einem dielektrischen Werkstoff getrennt sind, wobei diese Außenelektrode (8) mit der die elektrische Masse bildenden Form (1) in elektrisch leitendem Kontakt ist.

7. Form nach Anspruch 5 oder 6, **dadurch gekennzeichnet, dass** der kapazitive Fühler (4) außerdem eine Zwischenelektrode (12) (Hilfselektrode) umfasst, die zwischen die Mittelelektrode (7) und die Außenelektrode (8) eingesetzt ist.

8. Form nach Anspruch 7, **dadurch gekennzeichnet, dass** die Zwischenelektrode (12) (Hilfselektrode) so angeschlossen ist, dass sie auf einem Potential ist, das von der Mittelelektrode (7) und der Außenelektrode (8), zwischen die sie eingesetzt ist, verschieden ist.

9. Form nach einem der Ansprüche 5 bis 8, **dadurch gekennzeichnet, dass** der kapazitive Fühler (4) in einer Bohrung (13) der Wand (5) der Form (1) untergebracht und befestigt ist, die sich zur Formoberfläche (3) des Formhohlraums (2), wenn diese Oberfläche eben ist, oder zu der zur Formoberfläche (3) des Formhohlraums tangentialen Ebene, wenn diese Oberfläche gekrümmt ist, annähernd senkrecht erstreckt.

10. Form nach einem der Ansprüche 5 bis 9, **dadurch gekennzeichnet, dass** sie mehrere kapazitive Fühler (4) umfasst, die an verschiedenen Stellen der den Formhohlraum (2) begrenzenden Wand (5) angeordnet sind.

11. Form nach einem der Ansprüche 5 bis 10, **dadurch gekennzeichnet, dass** sie vom Typ Brieftaschenform ist, die mindestens zwei Schalenhälften umfasst, die durch Drehung um eine Drehachse um eine gemeinsame Achse voneinander entfernbar sind.

12. Form nach Anspruch 11, **dadurch gekennzeichnet, dass** jede Formhälfte mit mindestens einem kapazitiven Fühler (4) versehen ist.

13. Form nach Anspruch 11, **dadurch gekennzeichnet, dass** nur eine Formhälfte mit mindestens einem kapazitiven Fühler (4) versehen ist.

14. Form nach Anspruch 11, **dadurch gekennzeichnet, dass** sie außerdem einen von den Formhälften verschiedenen Formboden umfasst und dass der Formboden mit mindestens einem kapazitiven Fühler (4) versehen ist.

15. Form nach einem der Ansprüche 5 bis 14, **dadurch gekennzeichnet, dass** die Anschlussdrähte (14) des kapazitiven Fühlers (4) in eine in der Außenseite (16) der Form vorgesehene Nut (15) eingeführt sind und aus der Form (1) in ihrem unteren Teil oder in ihrem oberen Teil austreten.

16. Verfahren zur Herstellung einer Form nach einem der Ansprüche 5 bis 15, **dadurch gekennzeichnet, dass** es die folgenden Schritte umfasst:
- man bearbeitet in der Form (1) einen Vorformling (17) des Formhohlraums (2),
- man setzt einen kapazitiven Fühler (4) in eine Bohrung (13) ein, die in der Wand (5) der Form im Wesentlichen quer zur endgültigen Formoberfläche (3) des Formhohlraums (2) vorgesehen ist, und man befestigt ihn darin in seiner Montagestellung so, dass die Frontseite (19) des kapazitiven Fühlers an der endgültigen Formoberfläche (3) des Formhohlraums (2) nach innen vorsteht, und
- man beendet die Bearbeitung des Formhohlraums (2), indem man gleichzeitig den kapazitiven Fühler (4) so bearbeitet, dass dessen frontale Endseite (6) mit der Formoberfläche (3) des Formhohlraums (2) bündig ist und in der Formkontinuität dieser Oberfläche (3) ausgeformt ist.

17. Formungsanlage zur Herstellung von Behältern, insbesondere von Flaschen, durch Blasen oder Streckblasen von Vorformlingen aus Thermoplast, insbesondere aus PET, in einer Form (1), die mindestens einen Formhohlraum (2) umfasst,
**dadurch gekennzeichnet, dass** diese Anlage für die Durchführung des Messverfahrens nach einem der Ansprüche 1 bis 4 umfasst:
- mindestens eine Form, die gemäß einem der Ansprüche 5 bis 15 ausgebildet ist und einen kapazitiven Fühler (4) umfasst, der in die den Formhohlraum (2) begrenzenden Wand (5) der Form eingesetzt ist, wobei die Frontseite (6) des kapazitiven Fühlers (4) mit der Formoberfläche (3) des Formhohlraums (2) bündig ist und in der Formkontinuität dieser Oberfläche (3) ausgeformt ist, und
- Verarbeitungsmittel (23), um das Signal des kapazitiven Fühlers (4) zu verarbeiten und daraus eine für ein Merkmal des Behälters repräsentative Information abzuleiten.

18. Anlage nach Anspruch 17, **dadurch gekennzeichnet, dass** sie Mittel (24) zur Anzeige des erfassten Werts des Merkmals des Behälters gegenüber dem kapazitiven Fühler (4) umfasst.

19. Anlage nach Anspruch 17 oder 18, **dadurch gekennzeichnet, dass** sie Mittel (M) zur Speicherung des erfassten Werts des Merkmals des Behälters gegenüber dem kapazitiven Fühler (4) umfasst.

20. Anlage nach Anspruch 19, **dadurch gekennzeichnet, dass** die Speichermittel (M) geeignet sind, eine vorbestimmte Anzahl von Werten zu speichern, die von dem kapazitiven Fühler (4) bei nacheinander hergestellten Behältern erfasst wurden, und dass Mittel zur Analyse dieser vorbestimmten Anzahl von erfassten Werten vorgesehen sind, die geeignet sind, eine dauerhafte Änderung der erfassten Werte zu bestimmen, die für eine dauerhafte Änderung des Merkmals der nacheinander hergestellten Behälter repräsentativ ist.

21. Anlage nach Anspruch 20, **dadurch gekennzeichnet, dass** die Analysemittel mit einem Automaten (25) verbunden sind, der die Betriebsparameter der Anlage so steuert, dass diese Betriebsparameter in Abhängigkeit von der erfassten Änderung des Merkmals geändert werden.

22. Anlage nach einem der Ansprüche 17 bis 21, **dadurch gekennzeichnet, dass** mehrere kapazitive Fühler (4) an verschiedenen Stellen der den Formhohlraum (2) begrenzenden Wand (5) angeordnet sind.

23. Rotierende Formungsanlage vom Typ drehbares Karussell (26), umfassend mehrere auf dem Umfang angeordnete Formen, **dadurch gekennzeichnet, dass** sie gemäß einem der Ansprüche 17 bis 22 ausgebildet ist, wobei mindestens eine Form (1) mit mindestens einem kapazitiven Fühler (4) versehen ist.

24. Anlage nach einem der Ansprüche 17 bis 23, **dadurch gekennzeichnet, dass** das mit Hilfe mindestens eines kapazitiven Fühlers gemessene Merkmal die Dicke der Wand des Behälters gegenüber dem kapazitiven Fühler ist.
